# FASCICULE DE BREVET EUROPEEN

(11) **EP 2 491 972 B1**
(45) Date de publication et mention de la délivrance du brevet: **22.11.2017**
(21) Numéro de dépôt: 12152036.5
(22) Date de dépôt: 23.01.2012
(51) Int. Cl.: A61M 16/04, A61M 16/00

(54) **Appareil d'assistance respiratoire**
Gerät zur Atmungsunterstützung
Assisted breathing device

(30) Priorité: 23.02.2011 FR 1151455
(43) Date de publication de la demande: 29.08.2012
(73) Titulaire: Air Liquide Medical Systems, 92160 Antony (FR)
(72) Inventeur: Boulanger, Thierry, 37000 Tours (FR)
(74) Mandataire: De Cuenca, Emmanuel Jaime

(56) Documents cités:
- WO-A2-2010/046874
- DE-A1-102008 003 237
- SANDHU ET AL: "Measurement of endotracheal tube cuff leak to predict postextubation stridor and need for reintubation.", JOURNAL OF THE AMERICAN COLLEGE OF SURGEONS, vol. 190, no. 6, 1 juin 2000 (2000-06-01), pages 682-687, XP055007315, ISSN: 1072-7515
- M. MCD. FISHER ET AL: "The 'cuff-leak' test for extubation", ANAESTHESIA, vol. 47, no. 1, 1 janvier 1992 (1992-01-01), pages 10-12, XP055007312, ISSN: 0003-2409, DOI: 10.1111/j.1365-2044.1992.tb01943.x

## Description

La présente invention concerne un appareil d'assistance respiratoire.

L'invention concerne plus particulièrement un appareil d'assistance respiratoire comprenant un tube d'assistance destiné à être introduit dans la trachée d'un patient, un circuit de génération d'un flux de gaz respiratoire alimentant sélectivement ledit tube et une logique électronique de commande, le circuit de génération d'un flux étant piloté par la logique électronique, l'appareil comprenant en outre un ballonnet sélectivement gonflable par la logique électronique, le ballonnet étant monté autour du tube pour assurer le maintien du tube dans la trachée et empêcher un retour du gaz depuis la trachée, l'appareil comprenant un capteur de pression relié à la logique électronique pour mesurer sélectivement la pression dans le ballonnet et une interface de commande pour un utilisateur.

Les appareils d'assistance respiratoire utilisant un tube (ou sonde) muni d'un ballonnet gonflable (tel que par exemple décrit dans le document FR2638092) génèrent des tâches multiples pour les personnels hospitaliers.

Il est en effet nécessaire de contrôler la pression du ballonnet de la sonde d'intubation qui doit être maintenue à un niveau déterminé (généralement 20 à 30cm d'H2O).

Le contrôle de cette pression (et regonflage du ballonnet si besoin) doit être effectué par les infirmières plusieurs fois par jour pour chaque patient. Des publications estiment que cette pression du ballonnet est la plupart du temps très en dehors des recommandations [Intensive Care Med (2007) 33 :128-32]. Ceci a pour conséquence une augmentation des lésions trachéales (dues au surgonflage du ballonnet) [AM J Surg 1978 ; 135 : 452-7 *;* J Trauma 1997 ; 43 :741-7] ou une augmentation des infections (dues au sous-gonflage du ballonnet) [Am J Respir Crit Care Med 1996 ;154 :111-5]. WO2010/046874A2 divulgue les caractéristiques du préambule de la revendication 1. Lors d'une ventilation mécanique de longue durée, le patient peut développer un oedème laryngé (au niveau du ballonnet de la sonde d'intubation). Un tel oedème peut entraîner l'échec de l'extubation, c'est-à-dire l'échec du retrait du tube. Cet échec nécessite alors une nouvelle ré-intubation coûteuse. Certaines pratiques médicales se développent pour contrôler la présence ou non d'un oedème. Ces manipulations fastidieuses ne sont cependant pas réalisées de façon fiable et sécurisée.

La présente invention vise à proposer un appareil d'assistance respiratoire permettant d'améliorer ou d'aider le travail des personnels soignants.

Un but de la présente invention est ainsi de pallier tout ou partie des inconvénients de l'art antérieur relevés ci-dessus.

A cette fin, l'appareil selon l'invention, par ailleurs conforme à la définition générique qu'en donne le préambule ci-dessus, est essentiellement caractérisé en ce que la logique électronique est configurée pour réaliser sélectivement un test de pré-extubation en réponse à un actionnement via l'interface de commande, le test de pré-extubation comprenant la séquence suivante :
- une commande du dégonflement du ballonnet en dessous du seuil de gonflage déterminé,
- puis une mesure ou une détermination d'une éventuelle fuite de gaz en comparant les volumes de gaz entrant et sortant du tube,
- une génération d'une alarme si la fuite mesurée ou déterminée est inférieure à un seuil déterminé alors que le ballonnet est dégonflé.

Par ailleurs, des modes de réalisation de l'invention peuvent comporter l'une ou plusieurs des caractéristiques suivantes :
- la logique électronique est configurée pour commander le gonflage sélectif du ballonnet via le circuit de génération de gaz et via une vanne respective, notamment une électrovanne,
- la logique électronique est configurée pour mesurer ou déterminer une éventuelle fuite de gaz en comparant les volumes de gaz entrant et sortant du tube à partir d'une mesure de débit et/ou de pression dans le circuit de génération,
- le circuit de génération d'un flux de gaz respiratoire comprend, d'amont en aval, une arrivée de gaz sous pression, un régulateur de pression alimentant une première et une seconde vannes disposées en parallèle et connectables respectivement au tube et au ballonnet, le circuit de génération comprenant un unique capteur de pression disposé en aval de la seconde vanne, l'appareil comprenant un conduit de mesure de pression ayant une première extrémité reliée en aval du capteur de pression et une seconde extrémité connectable sélectivement soit au ballonnet pour assurer le gonflage contrôlé du ballonnet, soit à une prise de pression sur le tube pour mesurer une pression dans le tube,
- le circuit de génération d'un flux de gaz respiratoire comprend, d'amont en aval, une arrivée de gaz sous pression, un régulateur de pression alimentant une première et une seconde vanne disposées en parallèle et connectables respectivement au tube et au ballonnet, le circuit de génération comprenant un premier capteur de pression disposé en aval de la seconde vanne, l'appareil comprenant un conduit de mesure de pression ayant une première extrémité reliée en aval du premier capteur de pression et une seconde extrémité connectable sélectivement au ballonnet pour assurer le gonflage contrôlé du ballonnet, l'appareil comprenant un second capteur de pression relié à la logique électronique et à une prise de pression connectable, via un conduit de mesure de pression, à une prise de pression sur le tube pour mesurer une pression dans le tube,
- la logique électronique est configurée pour ouvrir la seconde électrovanne lorsque la seconde extrémité de la conduit de mesure de pression est connectée au ballonnet, pour assurer le gonflage contrôlé du ballonnet en fonction de la pression mesurée dans le ballonnet,
- la logique électronique de commande est configurée pour fermer la seconde électrovanne lorsque la seconde extrémité de la conduit de mesure de pression est connectée à la prise de pression sur le tube,
- la logique électronique est configurée pour réaliser le test de pré-extubation uniquement lorsque la seconde extrémité de la conduit (10) de mesure de pression est connectée à la prise de pression sur le tube,
- la première vanne est connectée au tube via un conduit de ventilation,
- l'appareil comprend une arrivée d'air provenant d'une source de gaz à haute pression, notamment d'une prise d'air murale et en ce qu'au moins l'une des vannes est une électrovanne,
- l'appareil une turbine pour générer le flux de gaz et en ce qu'au moins une des vannes est une vanne proportionnelle.

L'invention peut concerner également tout dispositif ou procédé alternatif comprenant toute combinaison des caractéristiques ci-dessus ou ci-dessous.

D'autres particularités et avantages apparaîtront à la lecture de la description ci-après, faite en référence aux figures dans lesquelles :
- la figure 1 représente de façon schématique et partielle, un premier exemple possible de structure et de fonctionnement d'un appareil d'assistance respiratoire selon l'invention,
- la figure 2 représente de façon schématique et partielle, un deuxième exemple possible de structure et de fonctionnement d'un appareil d'assistance respiratoire selon l'invention.

L'appareil d'assistance respiratoire représenté à la figure comprend un tube 2 d'assistance destiné à être introduit dans la trachée d'un patient et un circuit de génération d'un flux de gaz G respiratoire alimentant sélectivement ledit tube 2. Un ballonnet 6 sélectivement gonflable est disposé autour du tube 2. L'ensemble est piloté par une logique 8 électronique de commande via une interface 9 de commande pour un utilisateur.

Plus précisément, le circuit de génération d'un flux de gaz respiratoire comprend, d'amont en aval, une arrivée de gaz sous pression G, un régulateur 3 de pression alimentant une première et une seconde vannes 4, 7 disposées en parallèle. Les première 4 et seconde 7 vannes, qui sont de préférence des électrovannes, sont connectables respectivement au tube 2 et au ballonnet 6.

Un capteur 17 de pression est disposé en aval de la seconde électrovanne 7 pour mesurer une pression représentative de la pression dans le ballonnet 6.

L'appareil 1 comporte un conduit 10 de mesure de pression ayant une première extrémité reliée en aval du capteur 17 de pression et une seconde extrémité connectable sélectivement soit à une prise de pression du ballonnet 6 pour assurer le gonflage contrôlé du ballonnet 6, soit à une prise 11 de pression sur le tube 2 pour mesurer une pression dans le tube 2 (et représentative de la pression dan la trachée lorsque le tube 2 est relié aux voies respiratoires d'un patient).

La première électrovanne 4 est connectée au tube 2 via un conduit 5 de ventilation.

Selon une particularité de l'invention, la logique 8 électronique est configurée (par exemple programmée) pour offrir à l'utilisateur (personnel soignant) la possibilité de réaliser sélectivement un test de pré-extubation automatique et sécurisé en réponse à un actionnement via l'interface 9 de commande. Par exemple un bouton ou un choix dans un menu de l'appareil permet à l'utilisateur de déclencher ce test de pré-extubation.

Le test de pré-extubation mis en oeuvre par l'appareil comprend de préférence la séquence suivante :
- une commande du dégonflement du ballonnet 6 en dessous du seuil de gonflage déterminé,
- puis une mesure ou une détermination d'une éventuelle fuite de gaz en comparant les volumes de gaz entrant et sortant du tube 2,
- et, si la fuite mesurée ou déterminée est inférieure à un seuil déterminé alors que le ballonnet 6 est dégonflé, une génération d'une alarme ou d'un signal. En effet, l'absence de fuite alors que le ballonnet 6 est dégonflé indique qu'un oedème est susceptible de s'être formé dans la trachée du patient avec comme conséquence un possible échec de l'extubation. Il est alors possible de reporter cette extubation et de demander à l'appareil d'assurer un regonflage automatique du ballonnet 6).

La logique 8 électronique commande par exemple le dégonflement du ballonnet 6 en dessous du seuil de gonflage déterminé en fermant sélectivement la seconde électrovanne 7.

La mesure ou la détermination d'une éventuelle fuite de gaz peut être réalisée classiquement par la logique 8 électronique via des capteurs de pression/débit disposés dans le circuit de génération de gaz (non représentés par souci de simplification).

Lorsque le conduit 10 de mesure de pression est raccordé au ballonnet 6 (représenté par une ligne continue à la figure), l'appareil 1 peut assurer le maintien automatique de la pression du ballonnet 6 à la consigne réglée par l'utilisateur (par exemple 25 cm d'H2O). Ceci permet d'éviter les contrôles quotidiens.

Lorsque la logique 8 électronique commande la fermeture de la seconde électrovanne 8, la conduite 10 de mesure de pression peut être un moyen supplémentaire de mesure (via le capteur de pression 17). En particulier, la conduite 10 de mesure de pression peut être raccordée à une prise 11 de pression du tube 2 pour mesurer une pression oesophagienne (cf. la conduite représentée en traits discontinus).

L'architecture décrite permet d'offrir à l'utilisateur deux modes de fonctionnement. Ainsi, lorsque la seconde électrovanne 7 est fermée, l'appareil peut contrôler une pression externe, notamment celle dans le tube 2 ou celle du ballonnet 6. En revanche, lorsque cette seconde électrovanne 7 est ouverte, l'utilisateur peut utiliser la conduite 10 de mesure de pression pour contrôler et réguler le gonflage du ballonnet 6.

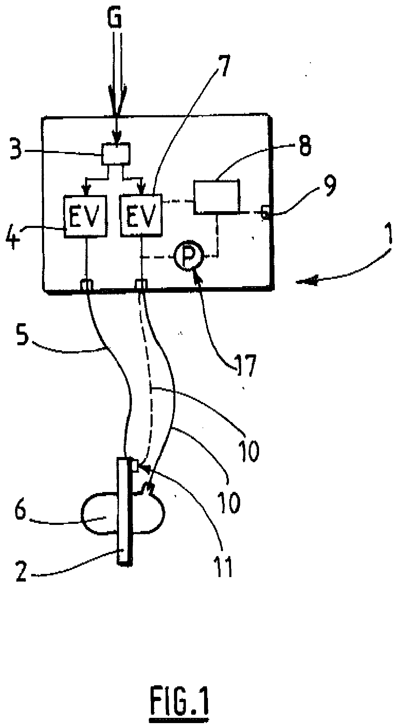

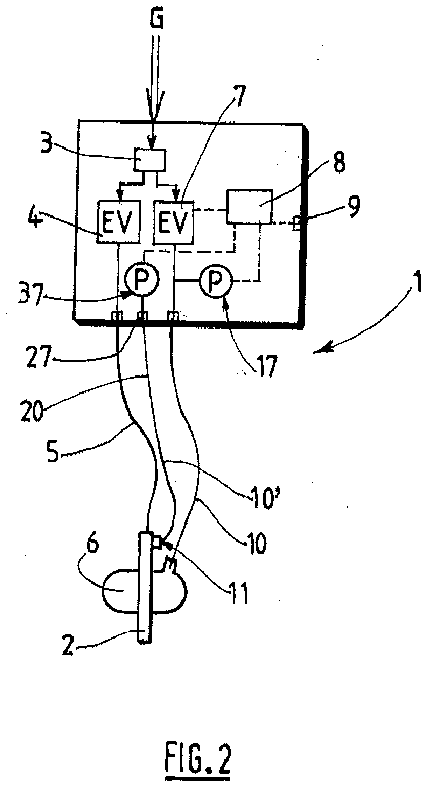

La figure 2 illustre une variante de réalisation qui se distingue de celle de la figure 1 uniquement en ce que l'appareil comprend un second capteur 37 de pression relié à la logique 8 électronique et à une prise 27 de pression connectable, via un conduit 20 de mesure de pression, à la prise de pression 11 sur le tube 2 pour mesurer une pression dans le tube 2. Les éléments identiques à ceux décrits précédemment sont désignés par les mêmes références numériques. Le premier capteur 17 de pression est quant à lui connecté au ballonnet 6 pour assurer le gonflage contrôlé du ballonnet 6.

Ainsi le second capteur 37 permet de mesurer directement la pression oesophagienne tout en conservant le pilotage du gonflage du ballonnet 6 via le premier capteur 17.

Par rapport à la variante de la figure 1, la réalisation de la figure 2 permet à l'utilisateur de disposer des deux fonctions simultanément.

Bien entendu l'invention n'est pas limitée aux exemples décrits ci-dessus. En particulier, la réalisation du test de pré-extubation peut être assurée par un appareil possédant une autre structure, par exemple du type de celle décrite dans le document FR2638092.

De même, l'invention peut s'appliquer aussi bien à des appareils classiques alimentés en air sous pression à partir d'une source (par exemple une prise murale d'hôpital) et muni d'électrovannes (comme représenté aux figures) qu'à des appareils comprenant une turbine pour produire l'air sous pression et munis vannes proportionnelles (en plus ou à la place des électrovannes).

## Revendications

1. Appareil d'assistance respiratoire comprenant un tube (2) d'assistance destiné à être introduit dans la trachée d'un patient, un circuit (3, 4, 5) de génération d'un flux de gaz (G) respiratoire alimentant sélectivement ledit tube (2) et une logique (8) électronique de commande, le circuit (3, 4, 5) de génération d'un flux étant piloté par la logique (8) électronique, l'appareil (1) comprenant en outre un ballonnet (6) sélectivement gonflable par la logique (8) électronique, le ballonnet (6) étant monté autour du tube (2) pour assurer le maintien du tube (2) dans la trachée et empêcher un retour du gaz depuis la trachée, l'appareil (1) comprenant un capteur (17) de pression relié à la logique (8) électronique pour mesurer sélectivement la pression dans le ballonnet (6) et une interface (9) de commande pour un utilisateur, **caractérisé en ce que** la logique (8) électronique est configurée pour réaliser sélectivement un test de pré-extubation en réponse à un actionnement via l'interface (9) de commande, le test de pré-extubation comprenant la séquence suivante :
- une commande du dégonflement du ballonnet (6) en dessous du seuil de gonflage déterminé,
- puis une mesure ou une détermination d'une éventuelle fuite de gaz en comparant les volumes de gaz entrant et sortant du tube (2),
- une génération d'une alarme si la fuite mesurée ou déterminée est inférieure à un seuil déterminé alors que le ballonnet (6) est dégonflé.

2. Appareil selon la revendication 1, **caractérisé en ce que** la logique (8) électronique est configurée pour commander le gonflage sélectif du ballonnet (6) via le circuit (3, 4, 5) de génération de gaz et via une vanne (7) respective, notamment une électrovanne.

3. Appareil selon la revendication 1 ou 2, **caractérisé en ce que** la logique (8) électronique est configurée pour mesurer ou déterminer une éventuelle fuite de gaz en comparant les volumes de gaz entrant et sortant du tube (2) à partir d'une mesure de débit et/ou de pression dans le circuit (3, 4, 5) de génération.

4. Appareil selon l'une quelconque des revendications 1 à 3, **caractérisé en ce que** le circuit (3, 4, 5) de génération d'un flux de gaz (G) respiratoire comprend, d'amont en aval, une arrivée de gaz sous pression (G), un régulateur (3) de pression alimentant une première et une seconde vannes (4, 7) disposées en parallèle et connectables respectivement au tube (2) et au ballonnet (6), le circuit (3, 4, 5) de génération comprenant un unique capteur (17) de pression disposé en aval de la seconde vanne (7), l'appareil (1) comprenant un conduit (10) de mesure de pression ayant une première extrémité reliée en aval du capteur (17) de pression et une seconde extrémité connectable sélectivement soit au ballonnet (6) pour assurer le gonflage contrôlé du ballonnet (6), soit à une prise (11) de pression sur le tube (2) pour mesurer une pression dans le tube (2).

5. Appareil selon l'une quelconque des revendications 1 à 3, **caractérisé en ce que** le circuit (3, 4, 5) de génération d'un flux de gaz (G) respiratoire comprend, d'amont en aval, une arrivée de gaz sous pression (G), un régulateur (3) de pression alimentant une première et une seconde vanne (4, 7) disposées en parallèle et connectables respectivement au tube (2) et au ballonnet (6), le circuit (3, 4, 5) de génération comprenant un premier capteur (17) de pression disposé en aval de la seconde vanne (7), l'appareil (1) comprenant un conduit (10) de mesure de pression ayant une première extrémité reliée en aval du premier capteur (17) de pression et une seconde extrémité connectable sélectivement au ballonnet (6) pour assurer le gonflage contrôlé du ballonnet (6), l'appareil comprenant un second capteur (37) de pression relié à la logique électronique et à une prise (27) de pression connectable, via un conduit (20) de mesure de pression, à une prise de pression (11) sur le tube (2) pour mesurer une pression dans le tube (2).

6. Appareil selon la revendication 4 ou 5, **caractérisé en ce que** la logique (8) électronique est configurée pour ouvrir la seconde électrovanne (7) lorsque la seconde extrémité de la conduit (10) de mesure de pression est connectée au ballonnet (6), pour assurer le gonflage contrôlé du ballonnet (6) en fonction de la pression mesurée dans le ballonnet (6).

7. Appareil selon l'une quelconque des revendications 4 à 6, **caractérisé en ce que** la logique (8) électronique de commande est configurée pour fermer la seconde électrovanne (7) lorsque la seconde extrémité de la conduit (10) de mesure de pression est connectée à la prise (11) de pression sur le tube (2).

8. Appareil selon l'une quelconque des revendications 4 à 7, **caractérisé en ce que** la logique (8) électronique est configurée pour réaliser le test de pré-extubation uniquement lorsque la seconde extrémité de la conduit (10) de mesure de pression est connectée à la prise (11) de pression sur le tube (2).

9. Appareil selon l'une quelconque des revendications 4 à 8, **caractérisé en ce que** la première vanne (4) est connectée au tube (2) via un conduit (5) de ventilation.

10. Appareil selon l'une quelconque des revendications 4 à 9, **caractérisé en ce que** l'appareil comprend une arrivée d'air provenant d'une source de gaz à haute pression, notamment d'une prise d'air murale et **en ce qu'**au moins l'une des vannes (4, 7) est une électrovanne.

11. Appareil selon l'une quelconque des revendications 4 à 9, **caractérisé en ce que** l'appareil comprend une turbine pour générer le flux de gaz et **en ce qu'**au moins une des vannes (4, 7) est une vanne proportionnelle.

## Patentansprüche

1. Gerät zur Atmungsunterstützung, umfassend ein Unterstützungsröhrchen (2), das dazu bestimmt ist, in die Luftröhre eines Patienten eingeführt zu werden, einen Kreis (3, 4, 5) zur Erzeugung eines Stroms von Atemgas (G), der das Röhrchen (2) selektiv speist, und eine elektronische Steuerlogik (8), wobei der Stromerzeugungskreis (3, 4, 5) von der elektronischen Logik (8) gesteuert wird, wobei das Gerät (1) des Weiteren einen durch die elektronische Logik (8) selektiv aufblasbaren Ballon (6) umfasst, wobei der Ballon (6) um das Röhrchen (2) herum angebracht ist, um den Halt des Röhrchens (2) in der Luftröhre sicherzustellen und ein Rückströmen des Gases aus der Luftröhre zu verhindern, wobei das Gerät (1) einen an die elektronische Logik (8) angeschlossenen Drucksensor (17) umfasst, um den Druck in dem Ballon (6) selektiv zu messen, und eine Steuerschnittstelle (9) für einen Benutzer, **dadurch gekennzeichnet, dass** die elektronische Logik (8) dazu ausgelegt ist, in Antwort auf eine Betätigung über die Steuerschnittstelle (9) selektiv einen Prä-Extubationstest auszuführen, wobei der Prä-Extubationstest die folgende Sequenz umfasst:
- einen Befehl zum Ablassen des Ballons (6) unter die bestimmte Aufblasgrenze,
- anschließend ein Messen oder Bestimmen eines eventuellen Gasverlusts durch Vergleichen der in das und aus dem Röhrchen (2) strömenden Gasvolumen,
- ein Erzeugen eines Alarms, wenn der gemessene oder bestimmte Verlust kleiner einer bestimmten Grenze ist, während der Ballon (6) abgelassen ist.

2. Gerät nach Anspruch 1, **dadurch gekennzeichnet, dass** die elektronische Logik (8) dazu ausgelegt ist, das selektive Aufblasen des Ballons (6) über den Gaserzeugungskreis (3, 4, 5) und über ein jeweiliges Ventil (7), insbesondere ein Magnetventil, zu steuern.

3. Gerät nach Anspruch 1 oder 2, **dadurch gekennzeichnet, dass** die elektronische Logik (8) dazu ausgelegt ist, einen eventuellen Gasverlust zu messen oder zu bestimmen durch Vergleichen der in das und aus dem Röhrchen (2) strömenden Gasvolumen anhand einer Durchfluss- und/oder Druckmessung in dem Erzeugungskreis (3, 4, 5).

4. Gerät nach einem der Ansprüche 1 bis 3, **dadurch gekennzeichnet, dass** der Kreis (3, 4, 5) zur Erzeugung eines Stroms von Atemgas (G) umfasst, von stromauf nach stromab, eine Zuführung von Druckgas (G), einen Druckregler (3), der ein erstes und ein zweites Ventil (4, 7) speist, die parallel angeordnet und jeweils mit dem Röhrchen (2) und dem Ballon (6) verbindbar sind, wobei der Erzeugungskreis (3, 4, 5) einen einzigen Drucksensor (17) umfasst, der stromab des zweiten Ventils (7) angeordnet ist, wobei das Gerät (1) eine Druckmessleitung (10) umfasst mit einem ersten Ende, das stromab des Drucksensors (17) angeschlossen ist, und einem zweiten Ende, das selektiv entweder mit dem Ballon (6) verbindbar ist, um das kontrollierte Aufblasen des Ballons (6) sicherzustellen, oder mit einem Druckaufnehmer (11) an dem Röhrchen (2), um einen Druck in dem Röhrchen (2) zu messen.

5. Gerät nach einem der Ansprüche 1 bis 3, **dadurch gekennzeichnet, dass** der Kreis (3, 4, 5) zur Erzeugung eines Stroms von Atemgas (G) umfasst, von stromauf nach stromab, eine Zuführung von Druckgas (G), einen Druckregler (3), der ein erstes und ein zweites Ventil (4, 7) speist, die parallel angeordnet und jeweils mit dem Röhrchen (2) und dem Ballon (6) verbindbar sind, wobei der Erzeugungskreis (3, 4, 5) einen ersten Drucksensor (17) umfasst, der stromab des zweiten Ventils (7) angeordnet ist, wobei das Gerät (1) eine Druckmessleitung (10) umfasst mit einem ersten Ende, das stromab des ersten Drucksensors (17) angeschlossen ist, und einem zweiten Ende, das selektiv mit dem Ballon (6) verbindbar ist, um das kontrollierte Aufblasen des Ballons (6) sicherzustellen, wobei das Gerät einen zweiten Drucksensor (37) umfasst, der an die elektronische Logik und an einen Druckaufnehmer (27) angeschlossen ist, der über eine Druckmessleitung (20) mit einem Druckaufnehmer (11) an dem Röhrchen (2) verbindbar ist, um einen Druck in dem Röhrchen (2) zu messen.

6. Gerät nach Anspruch 4 oder 5, **dadurch gekennzeichnet, dass** die elektronische Logik (8) dazu ausgelegt ist, das zweite Magnetventil (7) zu öffnen, wenn das zweite Ende der Druckmessleitung (10) mit dem Ballon (6) verbunden ist, um das kontrollierte Aufblasen des Ballons (6) abhängig von dem im Ballon (6) gemessenen Druck sicherzustellen.

7. Gerät nach einem der Ansprüche 4 bis 6, **dadurch gekennzeichnet, dass** die elektronische Steuerlogik (8) dazu ausgelegt ist, das zweite Magnetventil (7) zu schließen, wenn das zweite Ende der Druckmessleitung (10) mit dem Druckaufnehmer (11) an dem Röhrchen (2) verbunden ist.

8. Gerät nach einem der Ansprüche 4 bis 7, **dadurch gekennzeichnet, dass** die elektronische Logik (8) dazu ausgelegt ist, den Prä-Extubationstest nur dann auszuführen, wenn das zweite Ende der Druckmessleitung (10) mit dem Druckaufnehmer (11) an dem Röhrchen (2) verbunden ist.

9. Gerät nach einem der Ansprüche 4 bis 8, **dadurch gekennzeichnet, dass** das erste Ventil (4) über eine Beatmungsleitung (5) mit dem Röhrchen (2) verbunden ist.

10. Gerät nach einem der Ansprüche 4 bis 9, **dadurch gekennzeichnet, dass** das Gerät eine Luftzuführung umfasst, die von einer Hochdruckgasquelle, insbesondere einem Druckluft-Wandanschluss stammt, und dadurch, dass es sich bei mindestens einem der Ventile (4, 7) um ein Magnetventil handelt.

11. Gerät nach einem der Ansprüche 4 bis 9, **dadurch gekennzeichnet, dass** das Gerät eine Turbine umfasst, um den Gasstrom zu erzeugen, und dadurch, dass es sich bei mindestens einem der Ventile (4, 7) um ein Proportionalventil handelt.

## Claims

1. Assisted breathing device comprising an assistance tube (2) intended to be introduced into a patient's trachea, a circuit (3, 4, 5) for generating a breathing gas stream (G) selectively supplying said tube (2) and a control electronic logic (8), the circuit (3, 4, 5) for generating a stream being controlled by the electronic logic (8), the device (1) further comprising a balloon (6) suitable for being selectively inflated by the electronic logic (8), the balloon (6) being mounted about the tube (2) in order to secure the tube (2) in the trachea and prevent a return of the gas from the trachea, the device (1) comprising a pressure sensor (17) connected to the electronic logic (8) for selectively measuring the pressure in the balloon (6) and a control interface (9) for a user, **characterised in that** the electronic logic (8) is configured to selectively perform a pre-extubation test in response to an actuation via the control interface (9), the pre-extubation test comprising the following sequence:
- actuation of deflation of the balloon (6) below the defined inflation threshold,
- followed by measurement or determination of a potential gas leak by comparing the inflowing and outflowing gas volumes of the tube (2),
- generation of an alarm if the leak measured or determined is less than a defined threshold while the balloon (6) is deflated.

2. Device according to claim 1, **characterised in that** the electronic logic (8) is configured to actuate the selective inflation of the balloon (6) via the gas generation circuit (3, 4, 5) and via a respective valve (7), particularly an electrovalve.

3. Device according to claim 1 or 2, **characterised in that** the electronic logic (8) is configured to measure or determine a potential gas leak by comparing the inflowing and outflowing gas volumes of the tube (2) on the basis of a flow rate and/or pressure measurement in the generation circuit (3, 4, 5).

4. Device according to any one of claims 1 to 3, **characterised in that** the circuit (3, 4, 5) for generating a breathing gas stream (G) comprises, from upstream to downstream, an intake of pressurised gas (G), a pressure regulator (3) supplying a first and a second valves (4, 7) arranged in parallel and suitable for connection respectively to the tube (2) and to the balloon (6), the generation circuit (3, 4, 5) comprising a single pressure sensor (17) arranged downstream from the second valve (7), the device (1) comprising a pressure measurement conduit (10) having a first end connected downstream from the pressure sensor (17) and a second end suitable for selective connection either to the balloon (6) to ensure the controlled inflation of the balloon (6), or to a pressure point (11) on the tube (2) to measure a pressure in the tube (2).

5. Device according to any one of claims 1 to 3, **characterised in that** the circuit (3, 4, 5) for generating a breathing gas stream (G) comprises, from upstream to downstream, an intake of pressurised gas (G), a pressure regulator (3) supplying a first and a second valves (4, 7) arranged in parallel and suitable for connection respectively to the tube (2) and to the balloon (6), the generation circuit (3, 4, 5) comprising a first pressure sensor (17) arranged downstream from the second valve (7), the device (1) comprising a pressure measurement conduit (10) having a first end connected downstream from the first pressure sensor (17) and a second end suitable for selective connection to the balloon (6) to ensure the controlled inflation of the balloon (6), the device comprising a second pressure sensor (37) connected to the electronic logic and a pressure point (27) suitable for connection, via a pressure measurement conduit (20), to a pressure point (11) on the tube (2) to measure a pressure in the tube (2).

6. Device according to claim 4 or 5, **characterised in that** the electronic logic (8) is configured to open the second electrovalve (7) when the second end of the pressure measurement conduit (10) is connected to the balloon (6), to ensure the controlled inflation of the balloon (6) according to the pressure measured in the balloon (6).

7. Device according to any one of claims 4 to 6, **characterised in that** the control electronic logic (8) is configured to close the second electrovalve (7) when the second end of the pressure measurement conduit (10) is connected to the pressure point (11) on the tube (2).

8. Device according to any one of claims 4 to 7, **characterised in that** the electronic logic (8) is configured to carry out the pre-extubation test only when the second end of the pressure measurement conduit (10) is connected to the pressure point (11) on the tube (2).

9. Device according to any one of claims 4 to 8, **characterised in that** the first valve (4) is connected to the tube (2) via a ventilation conduit (5).

10. Device according to any one of claims 4 to 9, **characterised in that** the device comprise an air intake from a high-pressure gas source, particularly a wall-mounted air intake and **in that** at least one of the valves (4, 7) is an electrovalve.

11. Device according to any one of claims 4 to 9, **characterised in that** the device comprise a turbine for generating the gas stream and **in that** at least one of the valves (4, 7) is a proportional valve.
